# EUROPEAN PATENT APPLICATION

(11) **EP 4 755 875 A1**
(43) Date of publication of application: **10.06.2026**
(21) Application number: 24858606.7
(22) Date of filing: 28.08.2024
(51) Int. Cl.: C07D 241/44, A61K 31/502, A61P 9/10, A61P 25/00

(54) **PHTHALAZINONE COMPOUND AND PREPARATION METHOD THEREFOR, PHARMACEUTICAL COMPOSITION, AND USE**

(30) Priority: 31.08.2023 CN 202311121449
(71) Applicant: Beijing Wehand-Bio Pharmaceutical Co., Ltd, Beijing 102600 (CN); Institute of Materia Medica, Chinese Academy of Medical Sciences, Beijing 100050 (CN)
(72) Inventor: ZHANG, Peicheng, Beijing 102629 (CN); LIU, Zhihua, Beijing 102600 (CN); YUAN, Xiang, Beijing 102600 (CN); LIU, Yuanyuan, Beijing 102600 (CN); LI, Gangsheng, Beijing 102629 (CN); CHEN, Yanmin, Beijing 102600 (CN); YANG, Yanan, Beijing 102629 (CN); WANG, Tingting, Beijing 102600 (CN); WANG, Xujie, Beijing 102629 (CN); ZOU, Yuanyuan, Beijing 102600 (CN); ZHANG, Xu, Beijing 102629 (CN); JIANG, Jianshuang, Beijing 102629 (CN); FENG, Ziming, Beijing 102629 (CN); YU, Jinhao, Beijing 102629 (CN)
(74) Representative: Bandpay & Greuter
(86) International application number: PCT/CN2024/115048
(87) International publication number: WO 2025/045072

(57) **Abstract**

A phthalazinone compound or a pharmaceutically acceptable salt thereof, a pharmaceutical composition containing the compound, and a medical use of the compound. The compound of the present invention has a neuroprotective effect and can be used for treating/preventing cerebrovascular-related diseases.

## Description

### TECHNICAL FIELD

The present invention relates to a phthalazinone compound or a pharmaceutically acceptable salt thereof, and use thereof in preparation of a medicament for preventing and/or treating cerebrovascular-related diseases.

### BACKGROUND

Cerebrovascular diseases are a category of diseases that occur in the blood vessels of the brain and are characterized by the brain tissue injury resulting from vascular dysfunction, such as stroke. Acute cerebrovascular diseases are generally classified into two categories: ischemic and hemorrhagic types. Ischemic strokes account for 62.4% of new stroke cases. Currently, drugs for clinical treatment of ischemic strokes remain scarce. Although surgical thrombectomy allows for timely reperfusion of blood to the ischemic brain tissue, detrimental substances (such as inflammatory cytokines and excitatory amino acids) generated in the reperfusion process will cause damage to the blood-brain barrier and induce excessive immune-inflammatory responses. This further exacerbates pathological damage, leads to ischemia-reperfusion injury, and results in postoperative adverse effects such as hemorrhagic transformation, cerebral edema, and neuroinflammation, which directly affect post-stroke recovery. Therefore, it is of significant social importance to develop safe and effective novel drugs for treatment of stroke.

### SUMMARY

The present invention provides a phthalazinone compound or a pharmaceutically acceptable salt thereof, a pharmaceutical composition containing such a compound, and a medical use of such a compound. The compounds of the present invention have a neuroprotective effect and are useful for treatment/prevention of cerebrovascular-related diseases.

The present invention provides the following technical solutions:

According to the first aspect of the technical solution of the present invention, there is provided a compound represented by formula (I) or a pharmaceutically acceptable salt thereof: 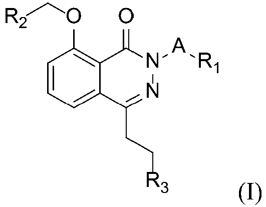 wherein:
A is -(CH₂)n-, wherein n is an integer of from 0 to 5;
R₁ is selected from hydrogen, halogen, cyano, optionally substituted alkyl, optionally substituted cycloalkyl, optionally substituted aryl, optionally substituted heterocycloalkyl, and optionally substituted heteroaryl;
R₂ is selected from hydrogen, cyano, optionally substituted alkyl, optionally substituted cycloalkyl, optionally substituted aryl, optionally substituted heterocycloalkyl, and optionally substituted heteroaryl; and
R₃ is optionally substituted C₁₋₄ linear or branched alkyl.

Optionally, said "optionally substituted" means unsubstituted or substituted with one or more substituents (preferably substituted with 1, 2, or 3 substituents), wherein the substituents in said "optionally substituted alkyl", "optionally substituted cycloalkyl", "optionally substituted aryl", "optionally substituted heterocycloalkyl", "optionally substituted heteroaryl", and "optionally substituted C₁₋₄ linear or branched alkyl" are each independently selected from halogen, hydroxyl, amino, carboxyl, and alkyl;
optionally, a halogen atom of said "halogen" is selected from fluorine, chlorine, bromine, and iodine;
optionally, when R₁ and R₂ are "optionally substituted alkyl", said alkyl is each independently C₁₋₁₀ linear or branched alkyl, optionally C₁₋₇ linear or branched alkyl, optionally C₁₋₅ linear or branched alkyl, optionally C₁₋₃ linear or branched alkyl, or optionally methyl, ethyl, n-propyl, isopropyl, n-butyl, isobutyl, tert-butyl, sec-butyl, n-pentyl, isopentyl, neopentyl, tert-pentyl, n-hexyl, isohexyl, heptyl, n-octyl, n-nonyl, or n-decyl;
optionally, said "cycloalkyl" is C₃-C₁₀ cycloalkyl having a monocyclic, fused, spiro or polycyclic structure, optionally C₃-C₇ monocyclic cycloalkyl, and optionally cyclopropyl, cyclobutyl, cyclopentyl, cyclohexyl or cycloheptyl;
optionally, said "aryl" is a 6- to 10-membered monocyclic or bicyclic fused aromatic ring group; optionally phenyl or naphthyl; and optionally phenyl, 1-naphthyl, or 2-naphthyl;
optionally, said "heterocycloalkyl" is 3- to 10-membered non-aromatic heterocyclyl containing 1, 2 or 3 heteroatoms selected from N, O and S on a ring; optionally, said heterocyclic ring is a 3- to 10-membered non-aromatic cyclic group containing 1 or 2 heteroatoms selected from N and O on a ring; optionally, said heterocyclic ring is a 3- to 6-membered non-aromatic cyclic group containing 1 or 2 heteroatoms selected from N and O on a ring; optionally, said heterocyclic ring is 3- to 10-membered non-aromatic cyclic group containing 1 or 2 heteroatoms selected from N and S on a ring; and optionally, said heterocyclic ring is 3- to 6-membered non-aromatic cyclic group containing 1 or 2 heteroatoms selected from N and S on a ring;
optionally, said "heteroaryl" is 5- to 10-membered heteroaryl containing 1 to 3 heteroatoms selected from N, O and S on a ring; optionally, said "heteroaryl" is 5- to 6-membered heteroaryl containing 1, 2 or 3 heteroatoms selected from N, O and S on a ring; optionally, said "heteroaryl" is selected from pyridyl, pyrroloyl, pyrazolyl, pyrimidinyl, pyrazinyl, pyridazinyl, thienyl, furyl, oxazolyl, isoxazolyl, thiazolyl, isothiazolyl, imidazolyl, triazolyl (1,2,4-triazolyl, 1,3,4-triazolyl or 1,2,3-triazolyl), thiadiazolyl (1,3,4-thiadiazolyl, 1,2,5-thiadiazolyl, 1,2,3-thiadiazolyl or 1,2,4-thiadiazolyl), and oxadiazolyl (1,3,4-oxadiazolyl, 1,2,5-oxadiazolyl, 1,2,3-oxadiazolyl or 1,2,4-oxadiazolyl); and optionally, said "heteroaryl" is selected from pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl, and pyrazin-3-yl.

Optionally, when R₃ is "C₁₋₄ linear or branched alkyl", said alkyl is optionally methyl, ethyl, propyl, isopropyl, n-butyl, or isobutyl.

Optionally, said n is 0, 1, 2, 3, 4 or 5.

Optionally, R₁ is selected from methyl, ethyl, n-propyl, isopropyl, n-butyl, isobutyl, tert-butyl, n-pentyl, isopentyl, cyclopropyl, cyclobutyl, cyclopentyl, cyclohexyl, phenyl or naphthyl, phenyl or naphthyl optionally substituted with 1, 2 or 3 halogens, pyridyl, pyrroloyl, pyrazolyl, pyrimidinyl, pyrazinyl, pyridazinyl, thienyl, furyl, oxazolyl, isoxazolyl, thiazolyl, isothiazolyl, and imidazolyl.

Optionally, R₂ is selected from methyl, ethyl, n-propyl, isopropyl, n-butyl, isobutyl, tert-butyl, n-pentyl, isopentyl, cyclopropyl, cyclobutyl, cyclopentyl, cyclohexyl, phenyl or naphthyl, and phenyl or naphthyl optionally substituted with 1, 2 or 3 halogens.

Optionally, R₃ is selected from methyl, ethyl, propyl, isopropyl, butyl, and isobutyl.

Optionally, the above-mentioned compound represented by formula (I) or the pharmaceutically acceptable salt thereof is selected from the following compounds:

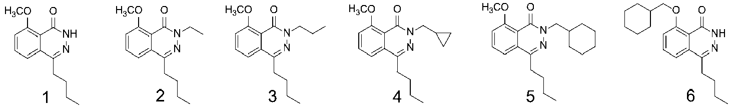

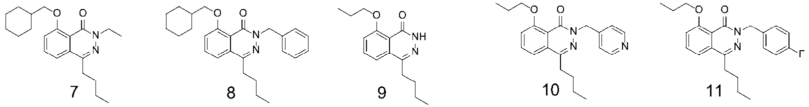

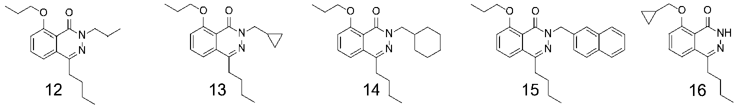

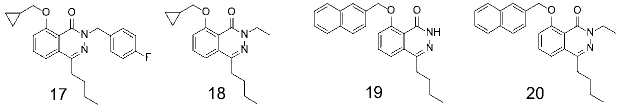

According to the second aspect of the technical solution of the present invention, there is provided a method for preparing the above-mentioned compound represented by formula (I) or the pharmaceutically acceptable salt thereof, comprising the following steps: 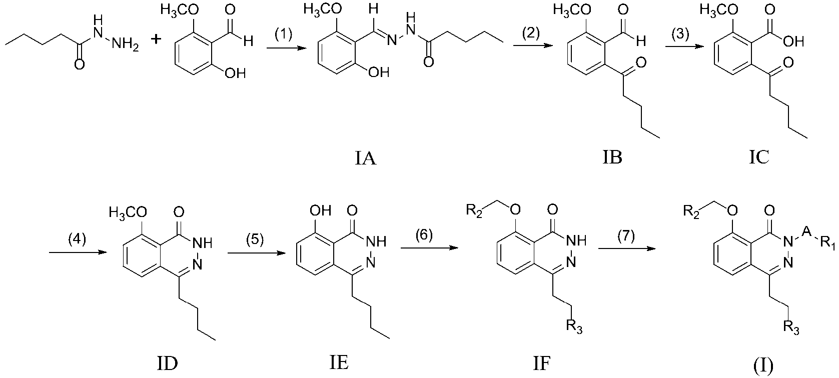
(1) preparing Compound IA via a condensation reaction of hydrazine valerate and 2-hydroxy-6-methoxybenzaldehyde as starting materials;
(2) preparing Compound IB from Compound IA via a transposition reaction;
(3) preparing Compound IC from Compound IB via an oxidation reaction;
(4) preparing Compound ID from Compound IC via a condensation reaction;
(5) preparing Compound IE via demethylation of Compound ID;
(6) preparing Compound IF by reacting Compound IE with a corresponding halogen; and
(7) preparing Compound (I) by reacting Compound IF with a corresponding halogen.

According to the third aspect of the technical solution of the present invention, there is provided a pharmaceutical composition comprising at least one compound represented by formula (I) or a pharmaceutically acceptable salt thereof, and optionally a pharmaceutically acceptable carrier and/or excipient;
preferably, the pharmaceutical composition further comprises one or more active pharmaceutical ingredients, in addition to the compound or the pharmaceutically acceptable salt thereof;
preferably, a formulation of the pharmaceutical composition may be in a liquid dosage form, a solid dosage form or a semi-solid dosage form.

Preferably, the liquid dosage form may be a solution (including a true solution and a colloidal solution), an emulsion (including o/w form, w/o form, and multiple emulsion), a suspension, an injection (including an aqueous injection, a powder injection, and an infusion), a nasal drop, etc.;
preferably, the solid dosage form may be a tablet (including a regular tablet, an enteric-coated tablet, a lozenge, a dispersible tablet, and an orally disintegrating tablet), a capsule (including a hard capsule, a soft capsule, and an enteric-coated capsule), a granule, a powder, a pellet, a dropping pill, a suppository, a film, a patch, a lyophilized powder injection, etc.; the semi-solid dosage form may be an ointment, a gel, a paste, etc.

The pharmaceutical composition may be formulated into ordinary formulations, sustained-release formulations, controlled-release formulations, targeting formulations, and various microparticle delivery systems.

Preferably, the additional active pharmaceutical ingredient is an active pharmaceutical ingredient that prevents and/or treats cerebrovascular-related diseases.

According to the fourth aspect of the technical solution of the present invention, there is provided use of the above-mentioned compound represented by formula (I) or the pharmaceutically acceptable salt thereof, or the above-mentioned pharmaceutical composition in preparation of a medicament for preventing and/or treating a cerebrovascular-related disease.

According to another aspect of the present invention, there is provided the above-mentioned compound represented by formula (I) or the pharmaceutically acceptable salt thereof, or the above-mentioned pharmaceutical composition for use in prevention and/or treatment of a cerebrovascular-related disease.

According to another aspect of the present invention, there is provided a medicament for preventing and/or treating a cerebrovascular-related disease, comprising the above-mentioned compound represented by formula (I) or the pharmaceutically acceptable salt thereof, or the above-mentioned pharmaceutical composition.

According to another aspect of the present invention, there is provided a method for treating a cerebrovascular-related disease, comprising administering, to a subject in need thereof, an effective amount of the above-mentioned compound represented by formula (I) or the pharmaceutically acceptable salt thereof, or the above-mentioned pharmaceutical composition.

Optionally, said cerebrovascular-related disease is selected from ischemic stroke, hemorrhagic stroke, and a post-stroke neurological recovery-related disease.

Some of the terms used in the present invention are defined below and other undefined terms will be understood to have the meanings known to those skilled in the art.

The term "optionally" means that the event or circumstance described subsequently may but does not have to occur, and the description includes the occasion where the event or circumstance occurs or the occasion where the event or circumstance does not occur. For example, the "alkyl optionally substituted with a halogen" means that the halogen may but does not have to be present, and the description includes the case where alkyl is substituted with halogen and the case where alkyl is not substituted with halogen.

The compounds according to the present invention include their pharmaceutically acceptable salts. The pharmaceutically acceptable salts refer to salts that are pharmaceutically acceptable and possess the desired pharmacological activity of the parent compound. The compounds according to the present invention may contain sufficient acidic groups, sufficient basic groups, or functional groups with both types, and accordingly react with some inorganic or organic bases, or inorganic and organic acids to form pharmaceutically acceptable salts. Examples of the pharmaceutically acceptable salt include sulfate, bisulfate, sulfite, bisulfite, phosphate, monohydrogen phosphate, dihydrogen phosphate, metaphosphate, pyrophosphate, hydrochloride, hydrobromide, hydroiodide, acetate, propionate, acrylate, formate, oxalate, malonate, succinate, fumarate, maleate, benzoate, chlorobenzoate, sulfonate, xylenesulfonate, phenylacetate, phenylpropionate, phenylbutyrate, citrate, lactate, γ-hydroxybutyrate, glycolate, and tartrate.

The formulations of the phthalazinone compounds or the pharmaceutical compositions containing the same according to the present invention are prepared according to the methods known to those skilled in the art. The excipients used in the manufacture of tablets, capsules, and coatings are conventional adjuvants, such as starch, gelatin, gum arabic, silica, and polyethylene glycol. Solvents for the liquid dosage form include, for example, water, ethanol, propylene glycol, and vegetable oils (such as corn oil, peanut oil, and olive oil). Formulations containing a compound of the present invention may also contain other adjuvants, such as surfactants, lubricants, disintegrants, preservatives, flavoring agents, and pigments.

To formulate the compounds of the present invention into tablets, a wide variety of excipients known in the art may be used, including diluents, binders, wetting agents, disintegrants, lubricants, and glidants. Diluents may be starch, dextrin, sucrose, glucose, lactose, mannitol, sorbitol, xylitol, microcrystalline cellulose, calcium sulfate, dicalcium phosphate, calcium carbonate, etc.; wetting agents may be water, ethanol, isopropanol, etc.; binders may be starch paste, dextrin, syrup, honey, glucose solution, microcrystalline cellulose, gum arabic paste, gelatin paste, sodium carboxymethyl cellulose, methyl cellulose, hydroxypropyl methyl cellulose, ethyl cellulose, acrylic acid resin, carbomer, polyvinylpyrrolidone, polyethylene glycol, etc.; disintegrants may be dry starch, microcrystalline cellulose, low-substituted hydroxypropyl cellulose, cross-linked polyvinylpyrrolidone, cross-linked sodium carboxymethyl cellulose, sodium carboxymethyl starch, sodium bicarbonate and citric acid, polyoxyethylene sorbitol fatty acid ester, sodium dodecyl sulfonate, etc.; lubricants and glidants may be talc, silica, stearate, tartaric acid, liquid paraffin, polyethylene glycol, etc.

Tablets may also be further formulated into coated tablets, such as sugar-coated tablets, film-coated tablets, enteric-coated tablets, or bilayer and multilayer tablets.

To formulate the unit dosage form into capsules, the active ingredient - a compound of the present invention - may be mixed with a diluent and a glidant, and the mixture is placed directly into hard or soft capsules. Alternatively, the active ingredient - a compound of the present invention - may be formed into granules or pellets together with a diluent, a binder and a disintegrant, and then placed into hard or soft capsules. The diluents, binders, wetting agents, disintegrants, and glidants used to prepare tablets of the compound of the present invention may also be used to prepare capsules of the compound of the present invention.

To formulate the compounds of the present invention into injections, water, ethanol, isopropanol, propylene glycol, or a mixture thereof may be used as a solvent, and appropriate amounts of solubilizers, co-solvents, pH adjusters, and osmotic pressure adjusters commonly used in the art are added. Solubilizers or co-solvents may be poloxamer, lecithin, hydroxypropyl-β-cyclodextrin, etc.; pH adjusters may be phosphates, acetates, hydrochloric acid, sodium hydroxide, etc.; osmotic pressure adjusters may be sodium chloride, mannitol, glucose, phosphates, acetates, etc. In case of preparing lyophilized powder injections, mannitol, glucose and the like may also be added as supporting agents.

To achieve the intended therapeutic purpose and enhance the therapeutic effect, the medicament or pharmaceutical composition of the present invention may be administered by any known route of administration.

The dose of administration of the pharmaceutical composition containing a compound of the present invention may vary widely depending on the nature and severity of the disease to be prevented or treated, the individual characteristics of the patient or animal, the route of administration, the dosage form, etc. Generally, a suitable daily dose range of the compound of the present invention is from 0.01 to 500 mg/kg of body weight, preferably 0.1 to 300 mg/kg of body weight. The above dose may be administered as a single dose unit or divided into multiple dose units, depending on the physician's clinical experience and the dosage regimen including application of other treatments.

The compound or composition of the present invention may be administered alone or in combination with other therapeutic or symptomatic drugs. When the compound of the present invention produces a synergistic effect with other therapeutic drugs, its dose should be adjusted as actually required.

### BRIEF DESCRIPTION OF THE DRAWINGS

FIG. 1 is a diagram showing the effect of Compound 17 on the survival rate of rats with ischemic stroke.
FIG. 2 is a diagram showing the effect of Compound 17 on the body weight of rats with ischemic stroke (n = 12, * *p* <0.05 vs. vehicle).
FIG. 3 is a diagram showing the effect of Compound 17 on the cerebral infarction volume in rats with ischemic stroke (n=12, ** p* <0.05 vs. vehicle, ** *p <0.01* vs. vehicle).

### DETAILED DESCRIPTION

Specific examples of the present invention are described below to further illustrate the technical solutions of the present invention, but the scope of the present invention is not limited thereto. Any alterations or equivalent substitutions without departing from the concept of the present invention are encompassed within the scope of the present invention.

### Preparation Methods

The compounds according to the present invention may be synthesized by the synthetic methods described herein and/or technologies well known in the art. For example, the compounds provided herein may be prepared by the general synthetic method below.

In a general synthetic method, the compound represented by formula (I) was prepared by the method.

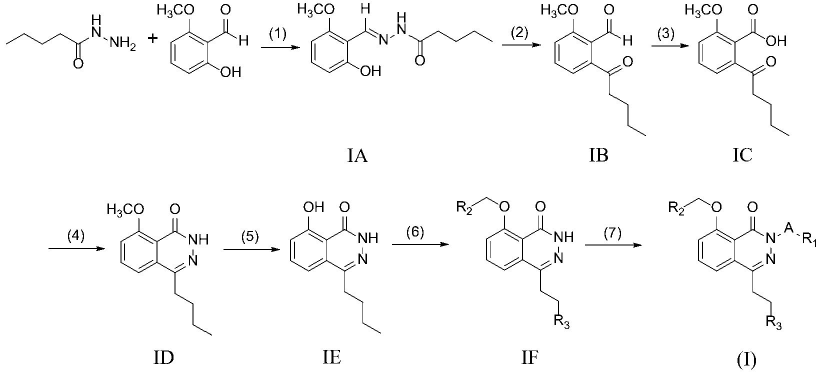

Specifically, the phthalazinone derivative according to the present invention could be prepared through a 6- or 7-step reaction in the method. For example, hydrazine valerate and 2-hydroxy-6-methoxybenzaldehyde as starting materials were subjected to a condensation reaction to prepare Compound IA, and then Compound IA was subjected to a transposition reaction to afford Compound IB; Compound IB was subjected to an oxidation reaction to afford Compound IC, and Compound IC was subjected to a condensation reaction to afford Compound ID; Compound ID was subjected to demethylation to prepare Compound IE; Compound IE was reacted to a corresponding halogen to afford Compound IF; and Compound IF was reacted to a corresponding halogen to afford the phthalazinone compound according to the present invention.

### Step 1:Synthesis of (E)-N'-(2-hydroxy-6-methoxybenzylidene)valerohydrazide

2-Hydroxy-6-methoxybenzaldehyde (27.0 g, 178 mmol) was dissolved into 150 mL of anhydrous ethanol, and heated at 80°C with stirring until the compound was completely dissolved. Valerohydrazide (21.6 g, 186 mmol) was dissolved into 150 mL of anhydrous ethanol and added to the reaction solution. The reaction mixture was stirred at 80°C for 2 h. The reaction solution was concentrated to afford a light yellow solid product (44.0 g, yield: 99%). The product could be used directly in the next reaction step without purification.

### Step 2: Synthesis of 2-methoxy-6-pentanoylbenzaldehyde

(*E*)-*N*'-(2-Hydroxy-6-methoxybenzylidene)valerohydrazide (44.0 g, 176 mmol) was dissolved into 500 mL of anhydrous THF. Lead tetraacetate (195 g, 440 mmol) was added in portions at 0°C. After reaction for 5 h, the reaction was monitored by TLC to ensure complete reaction. The reaction solution was subjected to suction filtration to remove the insoluble solids. 200 mL of water was added to quench the reaction. The reaction solution was extracted with ethyl acetate (300 mL×3). The organic layers were combined and then washed with saturated sodium bicarbonate (500 mL) and saturated brine (300 mL) respectively, dried over anhydrous sodium sulfate, filtered, and concentrated. The residue was purified by rapid column chromatography (silica gel, Hexane/EtOAc=10:1, v/v) to afford a colorless oily product (24.5 g, yield: 63%).

### Step 3: Synthesis of 2-methoxy-6-pentanoylbenzoic acid

2-Methoxy-6-pentanoylbenzaldehyde (24.5 g, 111 mmol) was dissolved into a mixed solution of 200 mL of acetonitrile and 100 mL of tert-butanol. Sodium chlorite (24.8 g, 222 mmol) was dissolved into 50 mL of water and slowly added into the reaction solution. Subsequently, an aqueous hydrogen peroxide solution (36%, 24.0 mL, 222 mmol) was slowly added dropwise into the reaction solution, and stirred overnight (10 h) at room temperature. The reaction was monitored by TLC to ensure complete reaction. The reaction solution was diluted with 100 mL of water and extracted with ethyl acetate (200 mL). The ethyl acetate phase was evaporated to dryness. Afterwards, the remaining aqueous layer was extracted with ethyl acetate (200 mL×2). The organic layers were combined and then washed with saturated brine (200 mL), dried over anhydrous sodium sulfate, filtered, and concentrated. The residue was purified by rapid column chromatography (silica gel, Hexane/EtOAc = 5:1, v/v) to afford a pair of isomers as a colorless oily product (16.4 g, yield: 67%).

### Step 4: Synthesis of 4-butyl-8-methoxy-phthalazin-1(2H)-one

2-Methoxy-6-pentanoylbenzoic acid (3.0 g, 12.7 mmol) and hydrazine hydrate (19.1 mmol, 0.96 mL) were charged into a reaction flask, and 50 mL of ethanol was added and reacted at 80°C for 2 h. The reaction was monitored by TLC to ensure complete reaction. The reaction solution was concentrated to afford a light yellow solid product (2.8 g, yield: 95%). The product could be used directly in the next reaction step without purification.

### Step 5: Synthesis of 4-butyl-8-hydroxy-phthalazin-1 (2H)-one

4-Butyl-8-methoxy-phthalazin-1(2H)-one (2.0 g, 8.6 mmol) was dissolved into 20 mL of toluene solution. AlCl₃ (3.4 g, 25.8 mmol) was slowly added to the reaction solution at 0°C and then reacted at 110°C for 40 min. The reaction was monitored by TLC to ensure complete reaction. The reaction solution was concentrated to remove the toluene, diluted with 50 mL of water, extracted with dichloromethane (50 mL×4), dried over anhydrous sodium sulfate, filtered, and concentrated to afford a light brown solid product (1.7 g, yield: 92%). The product could be used directly in the next reaction step without purification.

### Step 6: Synthesis of O-substituted phthalazinone derivative

2-Hydroxy-4-butyl-phthalazin-1(2H)-one (0.3 mmol) and halide (0.5 mmol) were charged into a reaction flask and dissolved by adding 3.0 mL of anhydrous DMF, and potassium carbonate (0.75 mmol) was added and reacted at 70°C for 1 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford the O-substituted phthalazinone derivative.

### Step 7: Synthesis of N,O-substituted phthalazinone derivative

O-Substituted phthalazinone derivative (0.3 mmol) and halide (0.5 mmol) were charged into a reaction flask and dissolved by adding 3.0 mL of anhydrous DMF. Sodium hydride (60%, 0.6 mmol) was added at 0°C and reacted at room temperature for 3 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford the N,O-substituted phthalazinone derivative.

### Example 1: 2-Ethyl-4-butyl-8-methoxy-phthalazin-1(2H)-one

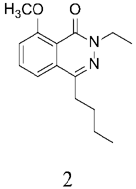

According to the procedure in Step 7 of Method I, 4-butyl-8-methoxy-phthalazine 1(2H)-one (0.3 mmol) as a starting material and bromoethane (0.5 mmol) were charged into a reaction flask and dissolved by adding 3.0 mL of anhydrous DMF. Sodium hydride (60%, 0.6 mmol) was added at 0°C, and reacted at room temperature for 3 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford the title compound 2.

ESI-MS: *m*/*z* 261.5 [M+H]⁺. ¹H NMR (500 MHz, DMSO-*d*₆) *δ*_{H}: 7.81 *(t, J =* 8.0 Hz, 1H), 7.43 (d, *J =* 8.0 Hz, 1H), 7.33 (d, *J =* 8.0 Hz, 1H), 4.05 (q, *J =* 6.5 Hz, 2H), 3.87 (s, 3H), 2.85 (t, *J =* 8.0 Hz, 2H), 1.59-1.65 (m, 2H), 1.33-1.41 (m, 2H), 1.23 (t, *J* = 6.5 Hz, 3H), 0.90 (t, *J =* 7.5 Hz, 3H). ¹³C NMR (125 MHz, DMSO-*d*₆) *δ*_{C}: 159.7, 156.0, 144.7, 134.4, 131.2, 116.3, 116.2, 113.3, 56.1, 45.1, 31.6, 29.7, 21.9, 13.8, 13.5.

### Example 2: 2-Propyl-4-butyl-8-methoxy-phthalazin-1(2H)-one

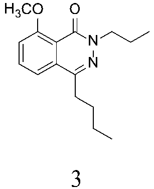

According to the procedure in Step 7 of Method I, 4-butyl-8-methoxy-phthalazine 1(2H)-one (0.3 mmol) as a starting material and iodopropane (0.5 mmol) were charged into a reaction flask, and dissolved by adding 3.0 mL of anhydrous DMF. Sodium hydride (60%, 0.6 mmol) was added at 0°C, and reacted at room temperature for 3 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 2-propyl-4-butyl-8-methoxy-phthalazin-1(2*H*)-one.

ESI-MS: m/z 275.3 [M+H]⁺.1H NMR (500 MHz, Methanol-*d*₄)*δ*_{H}: 7.78 (m, 1H), 7.42 (t, *J =* 7.6 Hz, 1H), 7.30 (dd, *J =* 8.9, 4.4 Hz, 1H), 4.09 (t, *J =* 7.2 Hz, 2H), 3.94 (d, *J =* 2.8 Hz, 3H), 2.88 (q, *J =* 7.7, 6.6 Hz, 2H), 1.81 (m, 2H),1.69 (m, 2H),1.42 (m, 2H), 0.94 (q, *J =* 7.3 Hz, 6H).¹³C NMR (125 MHz, Methanol-*d*₄) *δ*_{C}: 160.1, 158.3, 146.4, 134.4, 131.5, 116.2, 116.1, 112.8, 55.2, 52.3, 31.7, 29.7, 22.1, 21.4, 12. 9, 10.2.

### Example 3: 2-(Cyclopropylmethyl)-4-butyl-8-methoxy-phthalazin-1(2H)-one

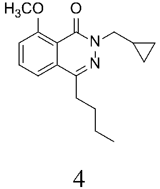

According to the procedure in Step 7 of Method I, 4-butyl-8-methoxy-phthalazine 1(2*H*)-one (0.3 mmol) as a starting material and bromomethylcyclopropane (0.5 mmol) were charged into a reaction flask, and dissolved by adding 3.0 mL of anhydrous DMF. Sodium hydride (60%, 0.6 mmol) was added at 0°C, and reacted at room temperature for 3 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 2-(cyclopropylmethyl)-4-butyl-8-methoxy-phthalazin-1(2*H*)-one.

ESI-MS: m/z 287.6 [M+H]⁺. ¹H NMR (500 MHz, Methanol-*d*₄) *δ*_{H}: 7.82 (t, *J*= 8.2 Hz, 1H), 7.48 (dd, *J =* 8.1, 0.9 Hz, 1H), 7.35 (d, *J =* 8.3 Hz, 1H), 4.02 (d, *J =* 7.1 Hz, 2H), 3.97 (s, 3H), 2.93 (m, 2H), 1.74 (m, 2H), 1.46 (m, 2H), 0.98 (t, *J =* 7.4 Hz, 3H), 0.87 (dd, *J =* 17.3, 6.9 Hz, 1H), 0.51 (m, 2H), 0.44 (m, 2H). ¹³C NMR (125 MHz, Methanol-*d*₄) *δ*_{C}: 161.5, 159.7, 147.7, 135.8, 133.1, 117.6, 117.5, 114.2, 56.8, 56.5, 33.1, 31.1, 23.5, 14.3, 11.3, 3.9.

### Example 4: 2-Cyclopropylmethyl-4-butyl-8-methoxy-phthalazin-1(2H)-one

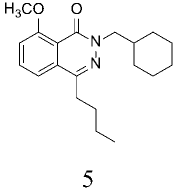

According to the procedure in Step 7 of Method I, 4-butyl-8-methoxy-phthalazine 1(2*H*)-one (0.3 mmol) as a starting material and bromomethylcyclopropane (0.5 mmol) were charged into a reaction flask, and dissolved by adding 3.0 mL of anhydrous DMF. Sodium hydride (60%, 0.6 mmol) was added at 0°C, and reacted at room temperature for 3 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 2-cyclopropylmethyl-4-butyl-8-methoxy-phthalazin-1(2H)-one.

ESI-MS: *m*/*z* 329.3 [M+H]⁺.1H NMR (500 MHz, DMSO-*d*₆) *δ*_{H}: 7.84 (td, *J =* 8.2, 1.5 Hz, 1H), 7.49 (dd, *J =* 8.0, 2.0 Hz, 1H), 7.36 (d, *J =* 8.3 Hz, 1H), 4.01 (d, *J =* 7.2 Hz, 2H), 3.97 (s, 3H), 2.94 (m, 2H), 2.00 (m, 1H), 1.74 (m, 4H), 1.66 (m, 3H), 1.45 (m, 2H), 1.26 (m, 2H), 0.98 (t, *J =* 7.4 Hz, 3H),0.98 (m, 3H).¹³C NMR (125 MHz, DMSO-*d*₆) *δ*_{C}: 161.6, 160.0, 147.6, 135.9, 132.9, 117.6, 117.5, 114.3, 57.8, 56.6, 38.5, 33.1, 31.8, 31.1, 27.6, 27.0, 23.4, 14.3.

### Example 5: 4-Butyl-8-(cyclohexylmethoxy)-phthalazin-1(2H)-one

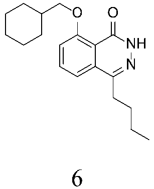

According to the procedure in Step 6 of Method I, 4-butyl-8-hydroxy-phthalazin-1(2*H*)-one (0.3 mmol) and (bromomethyl)cyclohexane (0.5 mmol) were charged into a reaction flask, and dissolved by adding 3.0 mL of anhydrous DMF. Potassium carbonate (0.75 mmol) was added and reacted at 70°C for 1 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 4-butyl-8-(cyclohexylmethoxy)-phthalazin-1(2*H*)-one.

ESI-MS: *m*/*z* 315.7 [M+H]⁺. ¹H NMR (500 MHz, DMSO-*d*₆) *δ*_{H}: 11.91(s, 1H), 7.78(t, *J* = 7.0 Hz, 1H), 7.42 (d, *J* = 7.0 Hz, 1H), 7.31 (d, *J =* 7.0 Hz, 1H), 3.89 (d, *J =* 7.5 Hz, 2H), 2.83 (t, *J =* 7.0 Hz, 2H),1.91 (m, 2H), 1.81 (m, 1H), 1.72 (m, 2H), 1.60-1.67 (overlap, 3H), 1.39 (m, 2H),1.07-1.30 (overlap, 5H), 0.92 (t, *J =* 7.5 Hz, 3H). ¹³C NMR (125 MHz, DMSO-*d*₆) *δ*_{C}: 159.3, 157.8, 144.8, 134.5, 131.7, 116.7, 116.2, 114.0, 73.8, 36.9, 31.5, 29.4, 29.0, 26.0, 25.3, 21.8, 14.0.

### Example 6: 2-Ethyl-4-butyl-8-(cyclohexylmethoxy)-phthalazin-1(2H)-one

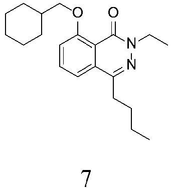

According to the procedure in Step 7 of Method I, 4-butyl-8-(cyclohexylmethoxy)-phthalazin-1(2H)-one (0.3 mmol) and bromoethane (0.5 mmol) were charged into a reaction flask, and dissolved by adding 3.0 mL of anhydrous DMF. Sodium hydride (60%, 0.6 mmol) was added at 0°C, and reacted at room temperature for 3 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 2-ethyl-4-butyl-8-(cyclohexylmethoxy)-phthalazin-1(2H)-one.

ESI-MS: m/z 343.6 [M+H]⁺. ¹H NMR (500 MHz, DMSO-*d*₆) *δ*_{H}: 7.76 (t, *J =* 8.0 Hz, 1H), 7.40 (d, *J =* 8.0 Hz,1H), 7.29 (d, *J =* 8.0 Hz, 1H), 4.04 (q, *J =* 7.0 Hz, 2H), 3.85 (d, *J =* 6.5 Hz, 2H), 2.83 (t, *J =* 7.0 Hz, 2H), 1.92 (m, 2H), 1.78 (m, 1H), 1.71 (m, 2H), 1.60-1.67 (overlap, 3H), 1.37(m, 2H), 1.19-1.30 (overlap, 5H), 1.15 (m, 1H), 1.08 (m, 2H), 0.90 (t, *J =* 7.5 Hz, 3H).¹³C NMR (125 MHz, DMSO-*d*₆) *δ*_{C}: 159.3, 156.1, 144.7, 134.3, 131.2, 116.3, 116.1, 114.1, 73.8, 45.0, 37.2, 31.6, 29.7, 29.2, 26.1, 25.4, 21.9, 13.8, 13.6.

### Example 7: 2-Benzyl-4-butyl-8-(cyclohexylmethoxy)-phthalazin-1(2H)-one

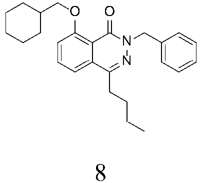

According to the procedure in Step 7 of Method **I**, 4-butyl-8-(cyclohexylmethoxy)-phthalazin-1(2*H*)-one (0.3 mmol) and benzyl bromide (0.5 mmol) were charged into a reaction flask, and dissolved by adding 3.0 mL of anhydrous DMF. Sodium hydride (60%, 0.6 mmol) was added at 0°C, and reacted at room temperature for 3 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 2-benzyl-4-butyl-8-(cyclohexylmethoxy)-phthalazin-1(2*H*)-one.

ESI-MS: *m*/*z* 405.9 [M+H]⁺. ¹H NMR (500 MHz, DMSO-*d*₆) *δ*_{H}: 7.79 (t, *J =* 8.0 Hz, 1H), 7.43 *(d, J=* 8.0 Hz, 1H), 7.22-7.33 (overlap, 6H), 5.22 (s, 2H), 3.86 *(d, J=* 6.5 Hz, 2H), 2.84 (t, *J =* 7.5 Hz, 2H), 1.92 (m, 2H), 1.78 (m, 1H), 1.70 (m, 2H), 1.60-1.66 (overlap, 3H), 1.36 (m, 2H), 1.14-1.30 (overlap, 3H), 1.06 (m, 2H), 0.88 (t, *J =* 7.5 Hz, 3H). ¹³C NMR (125 MHz, DMSO-*d*₆) *δ*_{C}: 159.4, 156.5, 145.1, 138.0, 134.6, 131.3, 128.4, 127.4, 127.1, 116.3, 116.3, 114.4, 73.8, 53.3, 37.1, 31.5, 29.6, 29.2, 26.1, 25.4, 21.8, 13.8.

### Example 8: 4-Butyl-8-propoxy-phthalazin-1(2H)-one

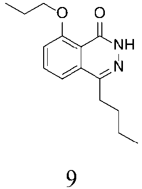

According to the procedure in Step 6 of Method I, 4-butyl-8-hydroxy-phthalazin-1(2H)-one (0.3 mmol) and 1-bromopropane (0.5 mmol) were charged into a reaction flask, and dissolved by adding 3.0 mL of anhydrous DMF. Potassium carbonate (0.75 mmol) was added and reacted at 70°C for 1 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 4-butyl-8-propoxy-phthalazin-1(2*H*)-one.

ESI-MS: *m*/*z* 261.5 [M+H]⁺. ¹H NMR (500 MHz, DMSO-*d*₆) *δ*_{H}: 12.02 (s, 1H), 7.75 (t, *J* = 7.5 Hz, 1H), 7.37 (d, *J =* 7.5 Hz, 1H), 7.27 (d, *J =* 7.5 Hz, 1H), 4.00 (t, *J =* 6.5 Hz, 2H), 2.78 (t, *J =* 7.5 Hz, 2H), 1.75 (m, 2H), 1.59 (m, 2H), 1.34 (m, 2H), 1.02 (t, *J =* 7.5 Hz, 3H), 0.87 (t, *J =* 7.5 Hz, 3H). ¹³C NMR (125 MHz, DMSO-*d*₆) *δ*_{C}: 159.2, 157.9, 144.9, 134.4, 131.7, 116.8, 116.3, 114.3, 70.1, 31.5, 29.5, 22.0, 22.0, 13.8, 10.5.

### Example 9: 2-(Pyridin-4-methylene)-4-butyl-8-propoxy-phthalazin-1(2H)-one

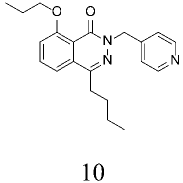

According to the procedure in Step 7 of Method **I**, 4-butyl-8-propoxy-phthalazin-1(2H)-one (0.3 mmol) and 4-(bromomethyl)-pyridine (0.5 mmol) were charged into a reaction flask, and dissolved by adding 3.0 mL of anhydrous DMF. Sodium hydride (60%, 0.6 mmol) was added at 0°C, and reacted at room temperature for 3 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 2-(pyridin-4-methylene)-4-butyl-8-propoxy-phthalazin-1(2*H*)-one.

ESI-MS: *m*/*z* 352.7 [M+H]⁺. ¹H NMR (500 MHz, DMSO-*d*₆) *δ*_{H}: 8.49 (d, *J* = 6.0 Hz, 2H), 7.83 (t, *J =* 8.0 Hz, 1H), 7.47 (d, *J =* 8.0 Hz, 1H), 7.36 (d, *J =* 8.0 Hz, 1H), 7.20 (d, *J =* 6.0 Hz, 2H), 5.24 (s, 2H), 4.03 (t, *J =* 6.5 Hz, 2H), 2.83 (t, *J=* 7.5 Hz, 2H), 1.76 (m, 2H), 1.64 (m, 2H), 1.35 (m, 2H), 1.02 (t, *J =* 7.5 Hz, 3H), 0.87 (t, *J =* 7.5 Hz, 3H). ¹³C NMR (125 MHz, DMSO-*d*₆) *δ*_{C}: 159.3, 156.5, 149.7, 146.8, 145.7, 134.7, 131.3, 122.1, 116.5, 116.1, 114.7, 70.2, 52.6, 31.5, 29.5, 22.0, 21.9, 13.7, 10.5.

### Example 10: 2-(4-Fluorobenzyl)-4-butyl-8-propoxy-phthalazin-1(2H)-one

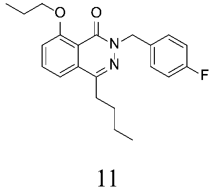

According to the procedure in Step 7 of Method **I**, 4-butyl-8-propoxy-phthalazin-1(2H)-one (0.3 mmol) and 4-fluorobenzyl bromide (0.5 mmol) were charged into a reaction flask, and dissolved by adding 3.0 mL of anhydrous DMF. Sodium hydride (60%, 0.6 mmol) was added at 0°C, and reacted at room temperature for 3 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 2-(4-fluorobenzyl)-4-butyl-8-propoxy-phthalazin-1(2*H*)-one.

ESI-MS: m/z 369.7 [M+H]⁺. ¹H NMR (500 MHz, DMSO-*d*₆) *δ*_{H}: 7.79 (t, *J =* 7.5 Hz, 1H), 7.43 (d, *J* = 7.5 Hz, 1H), 7.32-7.35 (overlap, 3H, H-7), 7.13 (m, 2H), 5.18 (s, 2H), 4.02 (t, *J =* 6.5 Hz, 2H), 2.84 (t, *J =* 7.5 Hz, 2H), 1.77 (m, 2H), 1.62 (m, 2H), 1.32 (m, 2H), 1.03 *(t, J=* 7.5 Hz, 3H), 0.88 (t, *J =* 7.5 Hz, 3H). ¹³C NMR (125 MHz, DMSO-*d*₆) *δ*_{C}: 162.4, 160.5, 159.3, 156.4, 145.2, 134.6, 134.1, 134.1, 131.3, 129.8, 129.7, 116.4, 116.3, 115.2, 115.1, 114.6, 70.2, 52.8, 31.5, 29.5, 22.1, 21.8, 13.8, 10.6.

### Example 11: 2-Propyl-4-butyl-8-propoxy-phthalazin-1(2H)-one

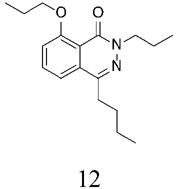

According to the procedure in Step 7 of Method **I**, 4-butyl-8-propoxy-phthalazin-1(2*H*)-one (0.3 mmol) and iodopropane (0.5 mmol) were charged into a reaction flask, and dissolved by adding 3.0 mL of anhydrous DMF. Sodium hydride (60%, 0.6 mmol) was added at 0°C, and reacted at room temperature for 3 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 2-propyl-4-butyl-8-propoxy-phthalazin-1(2*H*)-one.

ESI-MS: *m*/*z* 303.2 [M+H]⁺.1H NMR (500 MHz, Methanol-*d*₄) *δ*_{H}: 7.81 (dq, *J* = 24.4, 8.2, 7.8 Hz, 1H), 7.45 (dt, *J =* 22.2, 8.1 Hz, 1H), 7.34 (dt, *J =* 21.9, 8.0 Hz, 1H),4.06(m, 2H), 4.02 (d, *J =* 6.9 Hz, 1H),3.97 (m, 1H), 3.37 (dd, *J =* 12.7, 6.2 Hz, 1H), 2.86 (q, *J =* 10.2, 7.6 Hz, 1H), 1.82 (m, 2H), 1.76 (s, 3H),1.76 (m, 1H), 1.67 (dt, *J =* 24.4, 7.4 Hz, 2H), 1.39 (dd, *J =* 15.5, 8.1 Hz, 2H),1.10(m, 1H), 1.06 (d, *J =* 7.4 Hz, 1H), 0.92 (m, 6H).¹³C NMR (125 MHz, DMSO-*d*₆) *δ*_{C}: 159.3, 156.3, 144.5, 134.3, 131.2, 116.3, 116.2, 114.4, 70.2, 51.5, 31.5, 29.7, 22.1, 21.9, 21.4, 13.8, 11.1, 10.6.

### Example 12: 2-(Cyclopropylmethyl)-4-butyl-8-propoxy-phthalazin-1(2H)-one

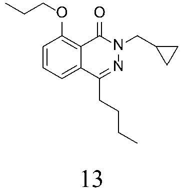

According to the procedure in Step 7 of Method I, 4-butyl-8-propoxy-phthalazin-1(2H)-one (0.3 mmol) and bromomethylcyclopropane (0.5 mmol) charged into a reaction flask, and dissolved by adding 3.0 mL of anhydrous DMF. Sodium hydride (60%, 0.6 mmol) was added at 0°C, and reacted at room temperature for 3 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 2-(cyclopropylmethyl)-4-butyl-8-propoxy-phthalazin-1(2*H*)-one.

ESI-MS: *m*/*z* 315.3 [M+H]⁺. ¹H NMR (500 MHz, DMSO-*d*₆) *δ*_{H}: 7.80 (t, *J =* 8.1 Hz, 1H), 7.43 (d, *J =* 8.0 Hz, 1H), 7.33 (d, *J =* 8.3 Hz, 1H), 4.04 (t, *J =* 6.3 Hz, 2H), 3.89 *(d, J=* 7.1 Hz, 2H), 2.86 (t, *J =* 7.6 Hz, 2H), 1.80 (m, 2H), 1.66 (m, 2H), 1.40 (m, 2H),1.27 (m, 1H), 1.06 (t, *J =* 7.4 Hz, 3H), 0.92 (t, *J =* 7.4 Hz, 3H), 0.45 (m, 2H), 0.37 (m, 2H). ¹³C NMR (125 MHz, DMSO-*d*₆) *δ*_{C}: 159.3, 156.4, 144.3, 134.3, 131.3, 116.4, 116.2, 114.4, 70.2, 54.4, 31.5, 29.5, 22.1, 21.9, 13.8, 10.6, 10.4, 3.2.

### Example 13: 2-(Cyclohexylmethyl)-4-butyl-8-propoxy-phthalazin-1(2H)-one

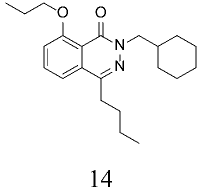

According to the procedure in Step 7 of Method **I,** 4-butyl-8-propoxy-phthalazin-1(2*H*)-one (0.3 mmol) and bromomethylcyclohexane (0.5 mmol) were charged into a reaction flask, and dissolved by adding 3.0 mL of anhydrous DMF. Sodium hydride (60%, 0.6 mmol) was added at 0°C, and reacted at room temperature for 3 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 2-(cyclohexylmethyl)-4-butyl-8-propoxy-phthalazin-1(2*H*)-one.

ESI-MS: m/z 357.4 [M+H]⁺.1H NMR (500 MHz, Methanol-*d*₄) *δ*_{H}: 7.79 (t, *J*= 8.2 Hz, 1H), 7.45 (d, *J =* 7.8 Hz, 1H), 7.32 (d, *J =* 8.3 Hz, 1H), 4.11 (t, *J =* 6.6 Hz, 2H), 3.99 (d, *J* = 7.3 Hz, 2H), 2.92 (t, *J =* 7.6 Hz, 2H), 1.99 (m, 1H), 1.93 (m, 2H), 1.73(m, 4H), 1.66 (dd, *J* = 10.6, 3.8 Hz, 3H), 1.44 (m, 2H), 1.24 (m, 2H), 1.22 (s, 1H), 1.12 (s, 1H), 1.10 (d, *J =* 7.5 Hz, 4H), 0.97 (t, *J =* 7.4 Hz, 3H).¹³C NMR (125 MHz, Methanol-*d*₄) *δ*_{C}: 161.2, 159.9, 147.5, 135.7, 132.9, 117.7, 117.4, 115.4, 72.1, 57.9, 38.4, 33.1, 31.8, 31.1, 27.6, 27.0, 23.4, 23.3, 14.3, 10.9.

### Example 14: 2-(Naphthylmethyl)-4-butyl-8-propoxy-phthalazin-1(2H)-one

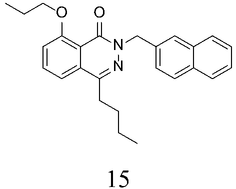

According to the procedure in Step 7 of Method I, 4-butyl-8-propoxy-phthalazin-1(2H)-one (0.3 mmol) and bromomethylnaphthalene (0.5 mmol) were charged into a reaction flask, and dissolved by adding 3.0 mL of anhydrous DMF. Sodium hydride (60%, 0.6 mmol) was added at 0°C, and reacted at room temperature for 3 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 2-(naphthylmethyl)-4-butyl-8-propoxy-phthalazin-1(2*H*)-one.

ESI-MS: *m*/*z* 401.3 [M+H]⁺.1H NMR (500 MHz, Methanol-*d*₄) *δ*_{H}: 7.78 (m, 5H), 7.52 (dd, *J* = 8.5, 1.8 Hz, 1H), 7.41 (m, 3H), 7.29 (d, *J* = 8.3 Hz, 1H), 5.47 (s, 2H), 4.08 (t, *J =* 6.7 Hz, 2H), 2.91 (t, *J =* 7.6 Hz, 2H), 1.91 (m, 2H), 1.71 (m, 2H), 1.38 (m, 2H), 1.08 (t, *J =* 7.4 Hz, 3H), 0.91 (t, *J =* 7.4 Hz, 3H).¹³C NMR (125 MHz, Methanol-*d*₄) *δ*_{C}: 161.2, 159.8, 148.3, 136.3, 135.9, 134.8, 134.3, 133.1, 129.2, 128.8, 128.6, 128.0, 127.2, 127.1, 126.9, 117.8, 117.5, 115.5, 72.1, 55.6, 33.1, 31.0, 23.4, 23.2, 14.3, 10.8.

### Example 15: 4-Butyl-8-(cyclopropylmethoxy)-phthalazin-1(2H)-one

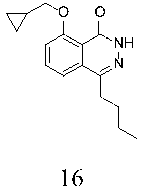

According to the procedure in Step 6 of Method I, 4-butyl-8-hydroxy-phthalazin-1(2H)-one (0.3 mmol) and (bromomethyl)cyclopropane (0.5 mmol) were charged into a reaction flask, and dissolved by adding 3.0 mL of anhydrous DMF. Potassium carbonate (0.75 mmol) was added and reacted at 70°C for 1 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 4-butyl-8-(cyclopropylmethoxy)-phthalazin-1 (2H)-one.

ESI-MS: m/z 273.5 [M+H]⁺. ¹H NMR (500 MHz, DMSO-*d*₆) *δ*_{H}: 12.03 (s, 1H), 7.77 (t, *J =* 8.0 Hz, 1H), 7.42 (d, *J =* 8.0 Hz, 1H), 7.30 (d, *J =* 8.0 Hz, 1H), 3.97 (d, *J =* 6.5 Hz, 2H), 2.81 (*t, J =* 7.0 Hz, 2H), 1.61 (m, 2H), 1.35 (m, 2H), 1.26 (m, 1H), 0.90 (t, *J =* 7.5 Hz, 3H), 0.54 (m, 2H), 0.40 (m, 2H). ¹³C NMR (125 MHz, DMSO-*d*₆) *δ*_{C}: 159.1, 157.9, 145.0, 134.4, 131.7, 117.0, 116.6, 115.1, 73.0, 31.5, 29.6, 22.0, 13.8, 10.1, 3.2.

### Example 16:

### 2-(4-Fluorobenzyl)-4-butyl-8-(cyclopropylmethoxy)-phthalazin-1(2H)-one

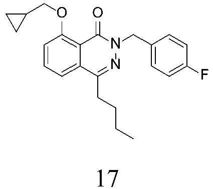

According to the procedure in Step 7 of Method **I,** 4-butyl-8-(cyclopropylmethoxy)-phthalazin-1(2*H*)-one (0.3 mmol) and 4-fluorobenzyl bromide (0.5 mmol) were charged into a reaction flask, and dissolved by adding 3.0 mL of anhydrous DMF. Sodium hydride (60%, 0.6 mmol) was added at 0°C, and reacted at room temperature for 3 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 2-(4-fluorobenzyl)-4-butyl-8-(cyclopropylmethoxy)-phthalazin-1(2H)-one.

ESI-MS: *m*/*z* 381.7 [M+H]⁺. ¹H NMR (500 MHz, DMSO-*d*₆) *δ*_{H}: 7.77 (t, *J* = 8.0 Hz, 1H), 7.42 (*d, J =* 8.0 Hz, 1H), 7.34 (m, 2H), 7.30 (d, *J* = 8.0 Hz, 1H), 7.13 (m, 2H), 5.18 (m, 2H), 3.95 (d, *J* = 7.5 Hz, 2H), 2.83 (t, *J* = 7.5 Hz, 2H), 1.61 (m, 2H), 1.31 (m, 2H), 1.25 (m, 1H), 0.87 (t, *J=* 7.5 Hz, 3H), 0.56 (m, 2H), 0.39 (m, 2H). ¹³C NMR (125 MHz, DMSO-*d*₆) *δ*_{C}: 162.4, 160.4, 159.2, 156.2, 145.2, 134.5, 134.1, 134.1, 131.3, 129.8, 129.7, 116.5, 116.4, 115.2, 115.2, 115.0, 73.0, 52.8, 31.5, 29.5, 21.8, 13.8, 10.0, 3.2.

### Example 17: 2-Ethyl-4-butyl-8-(cyclopropylmethoxy)-phthalazin-1(2H)-one

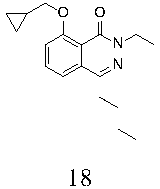

According to the procedure in Step 7 of Method **I**, 4-butyl-8-(cyclopropylmethoxy)-phthalazin-1(2*H*)-one (0.3 mmol) and bromoethane (0.5 mmol) were charged into a reaction flask, and dissolved by adding 3.0 mL of anhydrous DMF. Sodium hydride (60%, 0.6 mmol) was added at 0°C, and reacted at room temperature for 3 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 2-ethyl-4-butyl-8-(cyclopropylmethoxy)-phthalazin-1(2*H*)-one.

ESI-MS: *m*/*z* 301.7 [M+H]⁺. ¹H NMR (500 MHz, DMSO-*d*₆) *δ*_{H}: 7.77 (t, *J=* 8.0 Hz, 1H), 7.44 (d, *J =* 8.0 Hz, 1H), 7.31 (d, *J =* 8.0 Hz, 1H), 4.05 (q, *J =* 7.0 Hz, 2H), 3.97 (d, *J =* 6.0 Hz, 2H), 2.85 (t, *J =* 7.0 Hz, 2H), 1.64 (m, 2H), 1.38 (m, 2H), 1.22-1.30 (overlap, 4H), 0.91 (*t, J =* 7.5 Hz, 3H), 0.56 (m, 2H), 0.40 (m, 2H). ¹³C NMR (125 MHz, DMSO-*d*₆) *δ*_{C}: 159.1, 156.0, 144.8, 134.3, 131.2, 116.6, 116.5, 115.2, 73.0, 45.1, 31.6, 29.7, 21.9, 13.8, 13.5, 10.1, 3.1.

### Example 18: 4-Butyl-8-(naphthalene-2-methoxy)-phthalazin-1(2H)-one

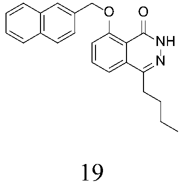

According to the procedure in Step 6 of Method **I,** 4-butyl-8-hydroxy-phthalazin-1(2H)-one (0.3 mmol) and 2-(bromomethyl)-naphthalene (0.5 mmol) were charged into a reaction flask, and dissolved by adding 3.0 mL of anhydrous DMF. Potassium carbonate (0.75 mmol) was added and reacted at 70°C for 1 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 4-butyl-8-(naphthalene-2-methoxy)-phthalazin-1(2H)-one.

ESI-MS: *m*/*z* 359.2 [M+H]⁺. ¹H NMR (500 MHz, DMSO-*d*₆) *δ*_{H}: 12.15 (s, 1H), 8.18 (s, 1H), 7.88-7.95 (overlap, 3H), 7.82 (t, *J =* 8.0 Hz, 1H), 7.72 (dd, *J =* 7.5 Hz, 1.5 Hz, 1H), 7.52 (m, 2H), 7.48 (m, 2H), 5.46 (m, 2H), 2.83 (t, *J =* 7.5 Hz, 2H), 1.64 (m, 2H), 1.39 (m, 2H), 0.91 (t, *J =* 7.5 Hz, 3H). ¹³C NMR (125 MHz, DMSO-*d*₆) *δ*_{C}: 158.5, 158.1, 145.1, 134.7, 134.5, 132.8, 132.5, 131.8, 127.9, 127.8, 127.7, 126.3, 126.0, 125.6, 125.2, 117.1, 117.0, 115.2, 70.1, 31.5, 29.6, 22.1, 13.9.

### Example 19: 2-Ethyl-4-butyl-8-(naphthalene-2-methoxy)-phthalazin-1(2H)-one

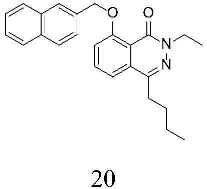

According to the procedure in Step 7 of Method I, 4-butyl-8-(naphthalene-2-methoxy)-phthalazin-1(2*H*)-one (0.3 mmol) and bromoethane (0.5 mmol) were charged into a reaction flask, and dissolved by adding 3.0 mL of anhydrous DMF. Sodium hydride (60%, 0.6 mmol) was added at 0°C, and reacted at room temperature for 3 h. The reaction was monitored by TLC to ensure complete reaction. 50 mL of water was added to quench the reaction. The reaction solution was extracted with ethyl acetate (50 mL×3). The organic layers were combined and then washed with saturated brine (20 mL), dried over anhydrous sodium sulfate, filtered, and concentrated. The residue was purified by rapid column chromatography to afford 2-ethyl-4-butyl-8-(naphthalene-2-methoxy)-phthalazin-1(2*H*)-one.

ESI-MS: m/z 387.6 [M+H]⁺. ¹H NMR (500 MHz, DMSO-*d*₆) *δ*_{H}: 8.14 (s, 1H), 7.95 (d, *J =* 7.5 Hz, 1H), 7.91 (m, 2H), 7.80 (t, *J* = 8.0 Hz, 1H), 7.76 (dd, *J* = 7.5 Hz, 1.5 Hz, 1H), 7.52 (m, 2H), 7.46 (m, 2H), 5.43 (s, 2H), 4.10 (q, *J=* 7.5 Hz, 2H), 2.85 (t, *J* = 7.5 Hz, 2H), 1.64 (m, 2H), 1.39 (m, 2H), 1.26 (t, *J =* 7.5 Hz, 3H), 0.91 (t, *J =* 7.5 Hz, 3H). ¹³C NMR (125 MHz, DMSO-*d*₆) *δ*_{C}: 158.6, 156.2, 144.8, 134.6, 134.3, 132.8, 132.5, 131.3, 128.0, 127.8, 127.7 126.3, 126.3, 126.0, 125.7, 125.3, 116.9, 116.8, 115.3, 70.3, 45.2, 31.6, 29.5, 21.8, 13.9, 13.6.

### Example 20: In Vitro Screening of Neuroprotective Activity

The *in vitro* neuroprotective models for evaluating the test compound were hydrogen peroxide injury and glutamate injury models. The PC-12 cells were seeded in a 96-well plate at a density of 1.5 × 10⁴ cells/mL, 100 µL per well. The experiment included a blank group, a model group, and drug treatment groups. The hydrogen peroxide injury and glutamate injury models were stimulated for 2 h with a final concentration of 120 µM hydrogen peroxide or 8 mM glutamate, respectively. Subsequently, the cells in the blank group were cultured in complete medium, whereas the cells in the drug treatment groups were cultured by adding 10 µM test compound. After 24-hour incubation for all groups, 10 µL of CCK-8 was added to each well. 60 min later, the absorbance at 490 nm was measured using a microplate reader. The viability was calculated. Improved Viability = (Viability in Drug Treatment Group - Viability in Model Group)/(Viability in Model Group). The results were shown in Tables 1 and 2.

**Table 1 Protective Effects of Phthalazinone Compounds on Nerve Cell Injury Induced by Hydrogen Peroxide (120 µM)**

| Compounds (10 µM) | Improved Viability (%) | Compounds (10 µM) | Improved Viability (%) |
|---|---|---|---|
| Edaravone | 11.28 ± 2.15 | 11 | 96.78 ± 8.64 |
| 2 | 35.81 ± 3.55 | 12 | 12.91 ± 6.03 |
| 4 | 23.37 ± 1.83 | 13 | 3.14 ± 1.45 |
| 5 | 61.89 ± 6.53 | 14 | 6.68 ± 3.51 |
| 7 | 0.81 ± 0.37 | 15 | 1.59 ± 1.13 |
| 8 | 12.06 ± 3.25 | 17 | 33.03 ± 2.37 |
| 9 | 60.19 ± 5.87 | 20 | 33.98 ± 3.92 |
| 10 | 1.93 ± 2.37 | | |

**Table 2 Protective Effects of Phthalazinone Compounds on Nerve Cell Injury Induced by Glutamate (8 mM)**

| Compounds (10 µM) | Improved Viability (%) | Compounds (10 M) | Improved Viability (%) |
|---|---|---|---|
| Edaravone | 3.14 ± 0.85 | 11 | 32.71 ± 6.21 |
| 2 | 0.04 ± 1.05 | 12 | 5.11 ± 2.33 |
| 4 | 9.75 ± 3.22 | 13 | 47.78 ± 8.14 |
| 5 | 13.99 ± 6.21 | 14 | 3.16 ± 3.54 |
| 7 | -3.69 ± 1.63 | 15 | -7.45 ± 4.12 |
| 8 | 7.99 ± 2.98 | 17 | 12.20 ± 2.57 |
| 9 | 7.48 ± 3.07 | 20 | 8.77 ± 3.01 |
| 10 | -5.60 ± 2.99 | | |

### Example 21: In Vivo Study on Anti-Ischemic Stroke Activity of Compound 17

Male SD rats weighing 280 - 300 g (Certificate No.: 11400700293705) were purchased from SPF (Beijing) Biotechnology Co., Ltd. (License No.: SYXK(Jing)2014-0023), and housed in the SPF-grade animal facility of the Institute of Materia Medica, Chinese Academy of Medical Science. The rats were fed standard maintenance diets and provided with food and water *ad libitum.* An appropriate amount of Compound 17 prepared in Example 16 was precisely weighed and dissolved into normal saline to prepare a 6 mg/mL drug solution for subsequent use. n-Butylphthalide (NBP) was prepared by the same method into a 20 mg/mL drug solution for subsequent use.

120 male SD rats (SPF grade) weighing 280 - 300 g were randomly divided into 6 groups: a Sham-operation group (Sham group), a model group (Vehicle group), a positive drug group (n-butylphthalide, NBP group), and drug treatment groups (low, medium, and high doses). There were 20 rats in each group. The improved middle cerebral artery occlusion method was employed, where the suture was slowly inserted from the right external carotid artery through the internal carotid artery into the proximal end of the anterior cerebral artery, occluding the blood supply to the contralateral side of the middle cerebral artery. In the Sham-operation group, the rats were subjected only to anesthesia and vascular dissection, without ligation of the blood vessel or insertion of the suture. In the drug treatment groups, the rats were administered by gavage with 10, 30 or 60 mg/kg of Compound 17, respectively. In the positive control group, the rats were administered by gavage with 200 mg/kg of NBP. In the model group, the rats were administered by gavage with the same volume of normal saline once daily for 7 consecutive days.

Three hours after the models were successfully constructed, the neurological function was scored on a 5-point scale according to the Longa scoring method: 0 point, no obvious neurological symptoms; 1 point, failure to fully straighten the contralateral forelimb; 2 points, circling to the contralateral side; 3 points, leaning or falling towards the contralateral side when walking; and 4 points, inability to walk on its own. Rats scoring 2 or 3 points were enrolled, while the others were excluded.

The volume of cerebral infarction in rats was determined by the 2,3,5-triphenyltetrazolium chloride staining method. 7 days after administration, the rats were deeply anesthetized and then euthanized by decapitation. The brains were removed and placed in a brain matrix. Afterwards, the brains were sectioned into 2-mm-thick slices along their coronal plane. The brain slices were immersed in a 2,3,5-triphenyltetrazolium chloride staining solution, and stained for 15 min in the dark. The brain slices were fixed overnight in 4% paraformaldehyde. Subsequently, the stained brain slices were scanned, and the volume of cerebral infarction was calculated.

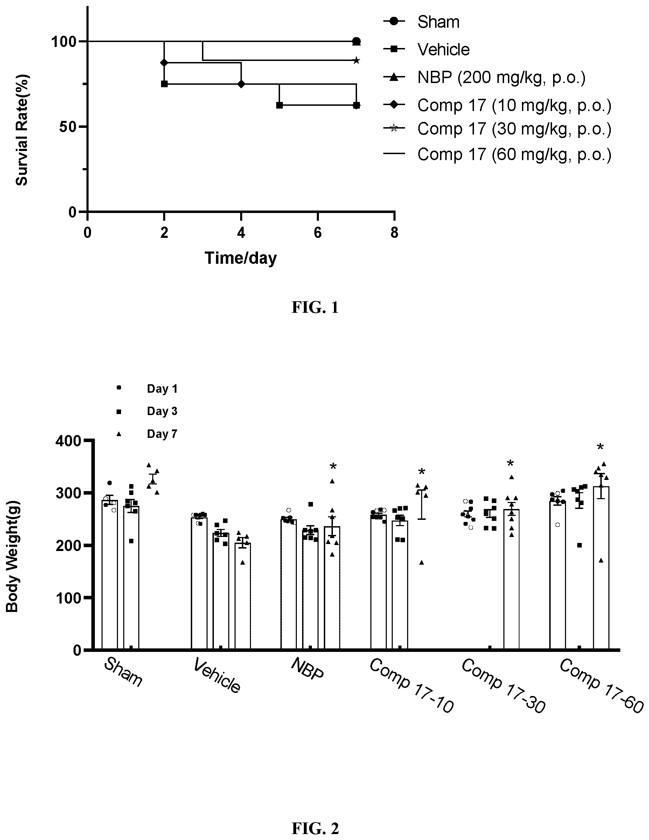

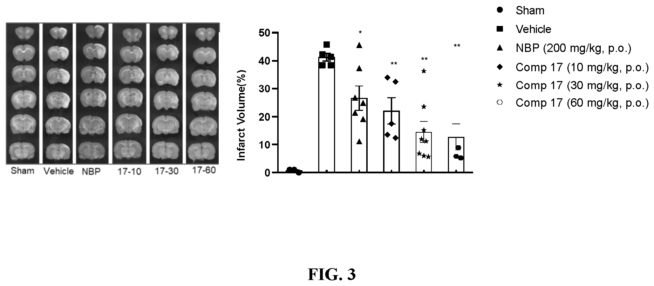

The experimental results were shown in FIGS. 1 to 3. It followed that intragastric administration of Compound 17 at different doses (10, 30, and 60 mg/kg) could effectively increase the survival rate of rats with ischemia-reperfusion injury, reverse the decrease trend in body weight of rats, and reduce the volume of cerebral infarction in a dose-dependent manner, and its effects were superior to those of the positive control drug butylphthalide (200 mg/kg).

## Claims

1. A compound represented by formula (I) or a pharmaceutically acceptable salt thereof: 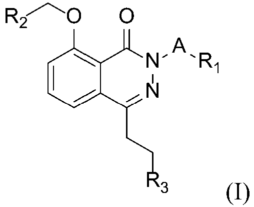 wherein:
A is -(CH₂)n-, wherein n is an integer of from 0 to 5;
R₁ is selected from hydrogen, halogen, cyano, optionally substituted alkyl, optionally substituted cycloalkyl, optionally substituted aryl, optionally substituted heterocycloalkyl, and optionally substituted heteroaryl;
R₂ is selected from hydrogen, cyano, optionally substituted alkyl, optionally substituted cycloalkyl, optionally substituted aryl, optionally substituted heterocycloalkyl, and optionally substituted heteroaryl; and
R₃ is optionally substituted C₁₋₄ linear or branched alkyl.

2. The compound represented by formula (I) or the pharmaceutically acceptable salt thereof according to claim 1, wherein
said "optionally substituted" means unsubstituted or substituted with one or more substituents (preferably substituted with 1, 2, or 3 substituents), wherein the substituents in said "optionally substituted alkyl", "optionally substituted cycloalkyl", "optionally substituted aryl", "optionally substituted heterocycloalkyl", "optionally substituted heteroaryl", and "optionally substituted C₁₋₄ linear or branched alkyl" are each independently selected from halogen, hydroxyl, amino, carboxyl, alkyl, and cyano;
optionally, a halogen atom of said "halogen" is selected from fluorine, chlorine, bromine, and iodine;
optionally, when R₁ and R₂ are "optionally substituted alkyl", said alkyl is each independently C₁₋₁₀ linear or branched alkyl, optionally C₁₋₇ linear or branched alkyl, optionally C₁₋₅ linear or branched alkyl, optionally C₁₋₃ linear or branched alkyl, or optionally methyl, ethyl, n-propyl, isopropyl, n-butyl, isobutyl, tert-butyl, sec-butyl, n-pentyl, isopentyl, neopentyl, tert-pentyl, n-hexyl, isohexyl, heptyl, n-octyl, n-nonyl, or n-decyl;
optionally, said "cycloalkyl" is C₃-C₁₀ cycloalkyl having a monocyclic, fused, spiro or polycyclic structure, optionally C₃-C₇ monocyclic cycloalkyl, and optionally cyclopropyl, cyclobutyl, cyclopentyl, cyclohexyl or cycloheptyl;
optionally, said "aryl" is a 6- to 10-membered monocyclic or bicyclic fused aromatic ring group; optionally phenyl or naphthyl; and optionally phenyl, 1-naphthyl, or 2-naphthyl;
optionally, said "heterocycloalkyl" is 3- to 10-membered non-aromatic heterocyclyl containing 1, 2 or 3 heteroatoms selected from N, O and S on a ring; optionally, said heterocyclic ring is 3- to 10-membered non-aromatic ring group containing 1 or 2 heteroatoms selected from N and O on a ring; optionally, said heterocyclic ring is 3- to 6-membered non-aromatic ring group containing 1 or 2 heteroatoms selected from N and O on a ring; optionally, said heterocyclic ring is 3- to 10-membered non-aromatic ring group containing 1 or 2 heteroatoms selected from N and S on a ring; and optionally, said heterocyclic ring is 3- to 6-membered non-aromatic ring group containing 1 or 2 heteroatoms selected from N and S on a ring;
optionally, said "heteroaryl" is 5- to 10-membered heteroaryl containing 1 to 3 heteroatoms selected from N, O and S on a ring; optionally, said "heteroaryl" is 5- to 6-membered heteroaryl containing 1, 2 or 3 heteroatoms selected from N, O and S on a ring; optionally, said "heteroaryl" is selected from pyridyl, pyrroloyl, pyrazolyl, pyrimidinyl, pyrazinyl, pyridazinyl, thienyl, furyl, oxazolyl, isoxazolyl, thiazolyl, isothiazolyl, imidazolyl, triazolyl (1,2,4-triazolyl, 1,3,4-triazolyl or 1,2,3-triazolyl), thiadiazolyl (1,3,4-thiadiazolyl, 1,2,5-thiadiazolyl, 1,2,3-thiadiazolyl or 1,2,4-thiadiazolyl), and oxadiazolyl (1,3,4-oxadiazolyl, 1,2,5-oxadiazolyl, 1,2,3-oxadiazolyl or 1,2,4-oxadiazolyl); and optionally, said "heteroaryl" is selected from pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl, and pyrazin-3-yl; and
optionally, when R₃ is "C₁₋₄ linear or branched alkyl", optionally said alkyl is methyl, ethyl, propyl, isopropyl, n-butyl, or isobutyl.

3. The compound represented by formula (I) or the pharmaceutically acceptable salt thereof according to claim 1 or 2, wherein said n is 0, 1, 2, 3, 4 or 5.

4. The compound represented by formula (I) or the pharmaceutically acceptable salt thereof according to any one of claims 1 to 3, wherein R₁ is selected from fluorine, chlorine, bromine, iodine, methyl, ethyl, n-propyl, isopropyl, n-butyl, isobutyl, tert-butyl, n-pentyl, isopentyl, cyclopropyl, cyclobutyl, cyclopentyl, cyclohexyl, phenyl or naphthyl, phenyl or naphthyl optionally substituted with 1, 2 or 3 halogens, pyridyl, pyrroloyl, pyrazolyl, pyrimidinyl, pyrazinyl, pyridazinyl, thienyl, furyl, oxazolyl, isoxazolyl, thiazolyl, isothiazolyl, and imidazolyl.

5. The compound represented by formula (I) or the pharmaceutically acceptable salt thereof according to any one of claims 1 to 4, wherein R₂ is selected from methyl, ethyl, n-propyl, isopropyl, n-butyl, isobutyl, tert-butyl, n-pentyl, isopentyl, cyclopropyl, cyclobutyl, cyclopentyl, cyclohexyl, phenyl or naphthyl, and phenyl or naphthyl optionally substituted with 1, 2 or 3 halogens.

6. The compound represented by formula (I) or the pharmaceutically acceptable salt thereof according to any one of claims 1 to 5, wherein R₃ is selected from methyl, ethyl, propyl, isopropyl, n-butyl, and isobutyl.

7. The compound represented by formula (I) or the pharmaceutically acceptable salt thereof according to any one of claims 1 to 6, wherein the compound is selected from the following compounds:

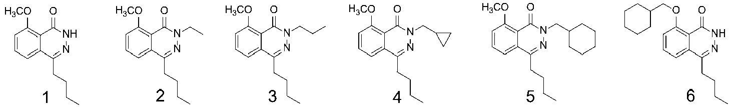

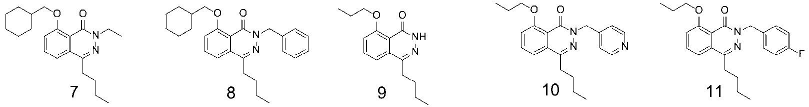

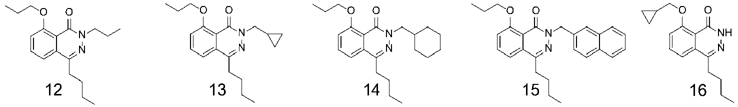

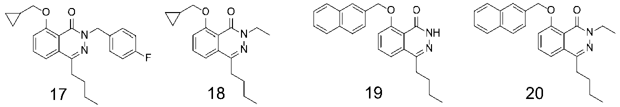

8. The compound represented by formula (I) or the pharmaceutically acceptable salt thereof according to any one of claims 1 to 7, wherein the pharmaceutically acceptable salt includes the following salts formed of the compound represented by formula (I) with acids: sulfate, bisulfate, sulfite, bisulfite, phosphate, monohydrogen phosphate, dihydrogen phosphate, metaphosphate, pyrophosphate, hydrochloride, hydrobromide, hydroiodide, acetate, propionate, acrylate, formate, oxalate, malonate, succinate, fumarate, maleate, benzoate, chlorobenzoate, sulfonate, xylenesulfonate, phenylacetate, phenylpropionate, phenylbutyrate, citrate, lactate, γ-hydroxybutyrate, glycolate, and tartrate.

9. A pharmaceutical composition, wherein the pharmaceutical composition comprises at least one compound represented by formula (I) or the pharmaceutically acceptable salt thereof according to any one of claims 1 to 8, and optionally a pharmaceutically acceptable carrier and/or excipient:
preferably, a formulation of the pharmaceutical composition is in a liquid dosage form, a solid dosage form or a semi-solid dosage form;
preferably, the liquid dosage form may be selected from a solution (including a true solution and a colloidal solution), an emulsion (including o/w form, w/o form, and multiple emulsion), a suspension, an injection (including an aqueous injection, a powder injection, and an infusion), and a nasal drop;
preferably, the solid dosage form is selected from a tablet (including a regular tablet, an enteric-coated tablet, a lozenge, a dispersible tablet, and an orally disintegrating tablet), a capsule (including a hard capsule, a soft capsule, and an enteric-coated capsule), a granule, a powder, a pellet, a dropping pill, a suppository, a film, a patch, and a lyophilized powder injection; and
preferably, the semi-solid dosage form is selected from an ointment, a gel, and a paste.

10. The pharmaceutical composition according to claim 9, wherein the pharmaceutical composition further comprises one or more additional active pharmaceutical ingredients in addition to the compound represented by formula (I) or the pharmaceutically acceptable salt thereof.

11. Use of the compound represented by formula (I) or the pharmaceutically acceptable salt thereof according to any one of claims 1 to 8, or the pharmaceutical composition according to claim 9 or 10 in preparation of a medicament for preventing and/or treating a cerebrovascular-related disease;
optionally, said cerebrovascular-related disease is selected from ischemic stroke, hemorrhagic stroke, and a post-stroke neurological recovery-related disease.

12. The compound represented by formula (I) or the pharmaceutically acceptable salt thereof according to any one of claims 1 to 8, or the pharmaceutical composition according to claim 9 or 10 for use in prevention and/or treatment of a cerebrovascular-related disease;
wherein said cerebrovascular-related disease is optionally selected from ischemic stroke, hemorrhagic stroke, and a post-stroke neurological recovery-related disease.

13. A medicament for preventing and/or treating a cerebrovascular-related disease, comprising the compound represented by formula (I) or the pharmaceutically acceptable salt thereof according to any one of claims 1 to 8, or the pharmaceutical composition according to claim 9 or 10,
wherein said cerebrovascular-related disease is optionally selected from ischemic stroke, hemorrhagic stroke, and a post-stroke neurological recovery-related disease.

14. A method for treating a cerebrovascular-related disease, comprising administering, to a subject in need thereof, an effective amount of the compound represented by formula (I) or the pharmaceutically acceptable salt thereof according to any one of claims 1 to 8, or the pharmaceutical composition according to claim 9 or 10,
wherein said cerebrovascular-related disease is optionally selected from ischemic stroke, hemorrhagic stroke, and a post-stroke neurological recovery-related disease.
